# FASCICULE DE BREVET EUROPEEN

(11) **EP 1 374 945 B1**
(45) Date de publication et mention de la délivrance du brevet: **31.08.2005**
(21) Numéro de dépôt: 03291493.9
(22) Date de dépôt: 19.06.2003
(51) Int. Cl.: A61N 1/05

(54) **Sonde coronaire comprenant des moyens perfectionnés de retenue**
Koronarsonde mit verbesserten Haltemitteln
Coronary probe with improved retaining means

(30) Priorité: 26.06.2002 FR 0207909
(43) Date de publication de la demande: 02.01.2004
(73) Titulaire: ELA MEDICAL, F-92541 Montrouge Cédex (FR)
(72) Inventeur: Ollivier, Jean-François, 91190 Villiers le Bacle (FR); Tacou, Jean-Michel, 77600 Conches sur Gondoire (FR)
(74) Mandataire: Dupuis-Latour, Dominique

(56) Documents cités:
- EP-A- 0 993 840
- WO-A-99/15228
- US-A- 4 550 737
- US-A- 6 006 122
- US-A- 6 136 021

## Description

L'invention concerne les sondes de stimulation cardiaque destinées à être implantées dans le réseau coronarien du coeur pour permettre la stimulation d'une cavité gauche par un "dispositif médical implantable actif" tel que défini par la directive 90/385/CEE du 20 juin 1990 du Conseil des communautés européennes, et plus précisément un dispositif tel qu'un stimulateur cardiaque, défibrillateur et/ou cardioverteur, notamment un stimulateur de type "multisite".

À la différence des cavités droites pour lesquelles il suffit d'implanter des sondes endocavitaires par le réseau veineux périphérique droit, pour stimuler les cavités gauches on introduit la sonde dans le réseau coronarien, par exemple avec une électrode disposée face au ventricule gauche, l'accès à l'entrée du sinus coronaire se faisant via l'oreillette droite.

La mise en place d'une telle sonde est une intervention particulièrement délicate, car la position des points de stimulation est très importante : ainsi, dans le cas d'un stimulateur de type "multisite", les deux points de stimulation ventricule gauche/ventricule droit doivent ainsi être les plus éloignés possible pour optimiser la resynchronisation de l'ensemble des cavités cardiaques. De la même façon, la stimulation de l'oreillette gauche impose un positionnement très précis de la sonde dans le réseau des veines coronaires. Bien entendu, lorsque le site choisi a été atteint, le maintien en position de la sonde doit pouvoir être assuré en toute sécurité à court et long terme, quelle que soit la taille de la veine.

L'invention vise plus précisément les moyens de retenue permettant d'assurer le maintien en position de l'extrémité de la sonde à l'emplacement du site de stimulation choisi dans le réseau coronaire.

Le EP-A-0 993 840 (ELA Médical) décrit une sonde coronaire pourvue de divers dispositifs élastiques susceptibles d'assurer cette fonction de retenue, par exemple au moyen d'une boule terminale, éventuellement gonflable, d'un chapelet de sphères de diamètres croissants, d'une jupe élastique, etc.

Ces divers moyens, s'ils sont efficaces pour assurer le maintien en place recherché, présentent une structure relativement complexe qui rend leur réalisation difficile ; au surplus, ils sont plutôt adaptés à des sondes à électrode sectorielle, étant conçus pour assurer une pression élastique entre la sonde et la paroi interne de la veine du côté opposé à celui où se trouve l'électrode sectorielle, de manière à accentuer la pression de cette électrode contre la paroi de la veine.

De plus, ces moyens de retenue rendent la sonde difficile à extraire ou à repositionner, sauf à prévoir des moyens relativement complexes pour assurer la réversibilité de l'implantation, par exemple en mettant en oeuvre des boules dégonflables ou des barbes retroussables.

En effet, une fois la sonde mise en place, il est souhaitable de pouvoir l'extraire ou bien la repositionner sans endommager les veines du réseau coronaire, ce qui impose pour le système de retenue une configuration peu traumatique.

Dans le même ordre d'idées, au moment de l'implantation, lors de la progression de la sonde, le chirurgien doit pouvoir franchir sans trop de difficultés des barrages tels que des valvules ou des embranchements du réseau coronaire : les moyens de rétention ne doivent pas, ou peu, gêner ces manoeuvres.

Enfin, les moyens de rétention doivent être conçus pour permettre l'introduction d'une sonde dans la lumière interne, de diamètre réduit, d'un cathéter-guide ainsi que l'utilisation d'un mandrin axial de guidage sur lequel pourra glisser la sonde (technique d'implantation des sondes dites "filoguidées");

L'un des buts de la présente invention est de proposer un moyen de retenue pour sonde coronaire qui pallie les divers inconvénients exposés ci-dessus, et qui soit notamment :
- de forme aussi peu traumatique que possible,
- compatible avec l'utilisation d'une sonde sectorielle ou non,
- compatible avec l'introduction dans un cathéter-guide de faible diamètre et l'enfilage sur un mandrin axial, et
- d'implantation entièrement réversible, de manière à permettre une extraction de la sonde sans endommagement de la veine.

L'un des buts de l'invention est aussi, et surtout, de proposer une sonde qui, pour un encombrement donné (imposé par le diamètre interne de la lumière du cathéter-guide) présente un pouvoir de rétention maximal, de manière que le chirurgien puisse être assuré que l'électrode qu'il a implantée sera maintenue en position sur le site de stimulation choisi, de façon précise et durable.

L'invention propose à cet effet, une sonde coronaire du type général décrit par le EP-A-0 993 840 précité, correspondant au préambule de la revendication 1.

De façon caractéristique de l'invention, la sonde comprend un relief hélicoïdal avec un filet s'enroulant autour du corps cylindrique.

Ce filet peut notamment :
- s'enrouler de façon non jointive autour du corps cylindrique ;
- s'enrouler autour du corps cylindrique sur deux à trois tours ;
- présenter un rayon nominal de filet variable, croissant puis décroissant sur la longueur d'enroulement ;
- présenter un rayon maximal de filet compris entre 0,75 et 1 fois le diamètre propre du corps cylindrique ;
- présenter un pas de filet constant ;
- être de profil arrondi.

Très avantageusement, le contour d'ensemble circulaire est excentré par rapport à l'axe du corps cylindrique avec une excentration comprise entre 15 et 25 % du diamètre propre du corps cylindrique.

Le diamètre du contour d'ensemble circulaire est de préférence compris entre 1,5 et 2 fois le diamètre propre du corps cylindrique.

On va maintenant décrire un exemple de réalisation de l'invention, en référence aux dessins annexés.
La figure 1 est une vue perspective de la sonde selon l'invention.
La figure 2 est une vue en élévation de la sonde de la figure 1, selon II-II de la figure 3,
La figure 3 est une vue de bout de cette même sonde, selon III-III de la figure 2.

Sur les figures, la référence 10 désigne la gaine souple de la sonde, dont seule l'extrémité distale, qui porte la tête de sonde est illustrée. Cette gaine 10 est creuse, de manière à pouvoir l'enfiler sur un mandrin axial de guidage, et elle loge un conducteur interne relié, côté distal, à l'électrode de la sonde et, côté proximal, à un connecteur de raccordement aux circuits électriques de stimulation et de recueil du dispositif médical implanté dans le corps du patient.

La gaine 10 est montée côté distal sur un élément de raccord 12, caractéristique de l'invention et qui sera décrit plus en détail par la suite, prolongé par un embout 14 formant la tête de sonde proprement dite, montée à l'extrémité distale de cet élément 12.

La tête de sonde 14 comporte, dans l'exemple illustré, un corps cylindrique 16 portant à son extrémité libre une électrode en forme de collier 20 en matériau conducteur (par exemple un carbone microporeux), disposée en retrait de l'extrémité du corps 16. Ce dernier est également pourvu d'une extrémité de forme approximativement hémisphérique 18 en élastomère de silicone contenant un stéroïde à libération progressive, permettant de minimiser localement la réaction inflammatoire et réduire l'élévation des seuils dans les premières semaines suivant l'implantation. Le silicone chargé par le stéroïde présente en outre la propriété avantageuse d'augmenter de volume en place par absorption.

La tête de sonde, tout comme la gaine 10 et l'élément de raccord 12, est creuse pour permettre l'enfilage d'un mandrin axial par un orifice axial 22 de la tête de sonde. Cet orifice 22 est normalement fermé par un joint mais peut être traversé de façon étanche par un mandrin d'angioplastie permettant le "filoguidage" de la sonde. Ce mandrin d'angioplastie, par exemple du type *595-J-014* de Cordis Corporation, est un mandrin très fin comportant une âme métallique surgainée d'un ressort et dont l'extrémité souple peut être introduite directement dans les vaisseaux sans risque de perforation. Ce mandrin, introduit dans la cavité centrale de la sonde, traverse l'ouverture étanche 22 et progresse ensuite à nu dans le réseau coronaire de façon à sélectionner plus aisément une veine collatérale. Une fois la veine sélectionnée, le chirurgien peut faire progresser le corps de sonde qu'il glisse sur le mandrin, dont le rôle sera celui d'un simple passe-fil de faible diamètre guidant axialement le corps de sonde.

On va maintenant décrire plus en détail la structure de l'élément de raccord 12, qui porte les moyens de retenue de l'extrémité de sonde dans la veine.

En effet, il est indispensable de pouvoir exercer une légère pression sur la veine afin d'établir un contact sûr et indépendant des mouvements cardiaques, en coinçant en place la tête de sonde de manière à éliminer tout risque de flottement dans la veine et tout retrait ou mouvement intempestif de la sonde.

L'élément 12 est formé, sur la majeure partie de sa longueur, d'un corps cylindrique 24, de même diamètre que la gaine 10 et raccordé à la tête de sonde, de diamètre inférieur, par une partie de transition tronconique 26. Le diamètre du corps cylindrique 24 et de la gaine 10 est par exemple de 1,6 mm, pour un diamètre de la tête de sonde 14 de 1,3 mm (ces indications n'étant, bien entendu, aucunement limitatives et données seulement à titre d'exemple).

De façon caractéristique de l'invention, le corps cylindrique 24 porte un relief en forme de filet hélicoïdal 28.

Ce filet présente un profil arrondi, non traumatique pour la veine avec laquelle il sera en contact, et s'étend à pas constant sur un nombre réduit de tours d'hélice, par exemple sur 2 tours ½ comme illustré sur le dessin, les tours étant de préférence non jointifs de manière à laisser subsister entre deux tours du filet un intervalle 30 dont la largeur correspond approximativement à la moitié de la largeur du pied du filet 28.

Avantageusement, le filet est un filet à rayon variable, d'abord croissant R₁, R₂ jusqu'à atteindre une valeur maximale R₃, puis décroissant R₄, R₅, de manière à présenter, d'une extrémité à l'autre du filet, une transition progressive avec la région centrale formant la saillie la plus marquée.

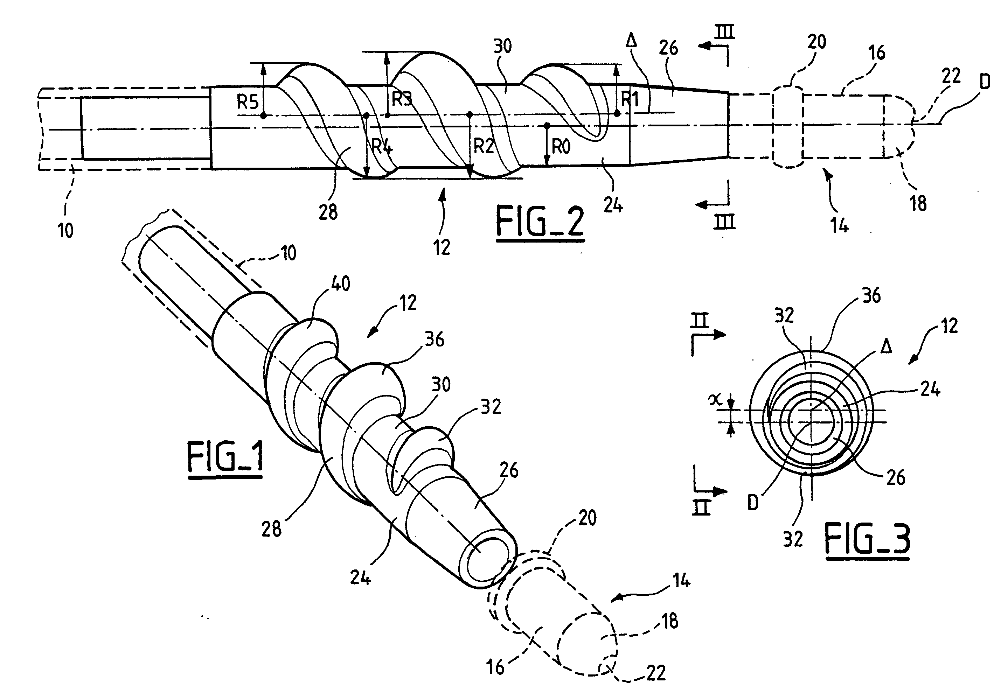

De plus, et de façon particulièrement avantageuse, l'axe Δ du filet est excentré par rapport à l'axe D du corps cylindrique 24 (figures 2 et 3). Cette excentration x est typiquement comprise entre 15 et 25 % du diamètre propre du corps cylindrique 24, par exemple une excentration de 0,3 mm pour un diamètre 2.R₀ = 1,6 mm.

Vu de bout (figure 3), le filet présente un contour circulaire et le rayon maximal R₃ du filet est choisi en fonction du diamètre de la lumière interne du cathéter-guide avec lequel la sonde sera utilisée de manière à occuper au maximum, sans frottement, l'espace interne de cette lumière. La dimension maximale R₃ du filet est par exemple choisie pour que le contour circulaire d'ensemble ait une dimension maximale R₂ + R₃ = 2,4 mm, cette dimension étant compatible avec un cathéter-guide de calibre 9 French (1 French = 0,33 mm) et de diamètre intérieur 2,45 mm.

L'excentration du filet par rapport à l'axe de la sonde permet d'augmenter au maximum la hauteur du filet 28 par rapport au corps cylindrique 24, et de ce fait améliore la qualité du vissage dans la veine ; la rétention de la sonde est alors maximale.

La présence de ce filet offre un avantage important en ce qui concerne la technique opératoire : en effet, lors de l'implantation, une fois la sonde arrivée en butée dans son déplacement en translation dans la veine, le chirurgien peut imprimer un mouvement additionnel de rotation au corps de sonde qui rend possible, par effet de vissage, la poursuite de la progression de la sonde sur quelques millimètres, avec renforcement corrélatif de l'ancrage de cette dernière dans la veine. Si nécessaire, pour augmenter le couple de vissage lors de cette manoeuvre, il est possible d'introduire dans le corps de sonde un mandrin pourvu d'un méplat entraînant directement en rotation l'extrémité de la sonde.

Le matériau de l'élément de raccord 12, pourvu du filet 28 formé mono-bloc par moulage sur le corps cylindrique 24, est avantageusement un élastomère de silicone, matériau peu traumatique et assurant une bonne biocompatibilité.

## Revendications

1. Une sonde coronaire, destinée à être implantée dans une veine du réseau coronarien pour la stimulation d'une cavité gauche du coeur, comprenant :
- une gaine creuse souple (10) logeant un conducteur interne,
- à l'extrémité distale de la gaine, un élément intermédiaire (12) avec un corps cylindrique (24) portant des moyens de retenue, et
- un embout formant tête de sonde (14), faisant saillie à l'extrémité distale de l'élément intermédiaire et pourvue d'au moins une électrode de stimulation (20) électriquement conductrice, reliée au conducteur interne et apte à venir en contact avec la paroi interne de la veine,
où lesdits moyens de retenue comprennent au moins un relief (28) formé sur le corps cylindrique (24) et présentant, en vue de bout, un contour d'ensemble circulaire et excentré par rapport à l'axe (D) du corps cylindrique (24), de manière à présenter localement un diamètre accru par rapport au diamètre propre du corps cylindrique,
sonde **caractérisée en ce que** ledit relief est un relief hélicoïdal avec un filet (28) s'enroulant autour du corps cylindrique.

2. La sonde de la revendication 1, où le filet s'enroule de façon non jointive autour du corps cylindrique.

3. La sonde de la revendication 1, où le filet s'enroule autour du corps cylindrique sur deux à trois tours.

4. La sonde de la revendication 1, où le rayon nominal du filet est un rayon variable, croissant puis décroissant sur la longueur d'enroulement.

5. La sonde de la revendication 1, où le pas du filet est un pas constant.

6. La sonde de la revendication 1, où le filet est un filet de profil arrondi.

7. La sonde de la revendication 1, où l'excentration (x) du contour est comprise entre 15 et 25 % du diamètre propre du corps cylindrique.

8. La sonde de la revendication 1, où le diamètre du contour d'ensemble circulaire est compris entre 1,5 et 2 fois le diamètre propre du corps cylindrique.

## Patentansprüche

1. Koronarsonde zum Einsetzen in eine Vene des Koronarnetzes zur Stimulierung einer linken Herzkammer, mit:
- einer einen inneren Leiter aufnehmenden hohlen flexiblen Hülse (10),
- am entfernten Ende der Hülse, einem Zwischenelement (12) mit einem Haltemittel tragenden zylindrischen Körper (24), und
- einem den Sondenkopf bildenden Endstück (14), welches vom entfernten Ende des Zwischenelementes hervorragt und mit mindestens einer elektrische leitenden Stimulationselektrode (20) versehen ist, welche mit dem inneren Leiter verbunden und dazu geeignet ist, mit der inneren Venenwand in Berührung zu treten,
wobei die Haltemittel mindestens ein auf dem zylindrischen Körper (24) gebildetes Relief (28) umfassen, welches in Draufsicht einen kreisförmigen und in Bezug zur Achse (D) des zylindrischen Körpers (24) exzentrischen Gesamtumfang aufweist, um so örtlich einen erhöhten Durchmesser in Bezug auf den Eigendurchmesser des zylindrischen Körpers aufzuweisen,
**dadurch gekennzeichnet, dass** das Relief ein schraubenförmiges Relief mit einem sich um den zylindrischen Körper windenden Gang (28) ist.

2. Sonde gemäß Anspruch 1, wobei der Gang sich in sich nicht verbindender Weise um den zylindrischen Körper windet.

3. Sonde gemäß Anspruch 1, wobei der Gang sich durch zwei bis drei Umdrehungen um den zylindrischen Körper windet.

4. Sonde gemäß Anspruch 1, wobei der Nenndurchmesser des Ganges ein variabler Durchmesser ist, der entlang der Windungslänge ansteigt und dann abnimmt.

5. Sonde gemäß Anspruch 1, wobei die Gangsteigung eine konstante Steigung ist.

6. Sonde gemäß Anspruch 1, wobei der Gang ein Gang mit einem abgerundeten Umfang ist.

7. Sonde gemäß Anspruch 1, wobei die Exzentrizität (x) des Umfangs zwischen 15 und 25 % des Eigendurchmessers des zylindrischen Körpers beträgt.

8. Sonde gemäß Anspruch 1, wobei der Durchmesser des kreisförmigen Gesamtumfangs zwischen 1,5- und 2-mal dem Eigendurchmesser des zylindrischen Körpers beträgt.

## Claims

1. A coronary probe, intended for implantation in a vein of the coronary network for the stimulation of a left cavity of the heart, comprising:
- a flexible hollow sheath (10) having in it an internal conductor,
- at the distal end of the sheath, an intermediate element (12) having a cylindrical body (24) supporting a retention means,
- a tip forming a probe head (14), protruding at the distal end of the intermediate element and provided with at least one electrically conducting stimulation electrode (20), connected to the internal conductor and adapted for abutting the inner wall of the vein,
wherein said retention means comprises at least one protrusion (28) formed on the cylindrical body (24) and having, as viewed from an end, an overall contour which is circular and offset with respect to the axis (D) of the cylindrical body (24) so as to have locally a diameter that is increased as compared to the intrinsic diameter of the cylindrical body,
said probe being **characterised in that** said protrusion is an helical protrusion with a thread (28) extending around the cylindrical body.

2. The probe of claim 1, wherein the thread extends around the cylindrical body in a spaced manner.

3. The probe of claim 1, wherein the thread extends around the cylindrical body over two to three turns.

4. The probe of claim 1, wherein the nominal radius of the thread is a variable radius that increases then decreases over the extension length.

5. The probe of claim 1, wherein the pitch of the thread is a constant pitch.

6. The probe of claim 1, wherein the thread is a round-profile thread.

7. The probe of claim 1, wherein the offset (x) of the contour is between 15 and 25 % of the intrinsic diameter of the cylindrical body.

8. The probe of claim 1, wherein the diameter of the overall circular contour is between 1.5 and 2 times the intrinsic diameter of the cylindrical body.
